# EUROPEAN PATENT SPECIFICATION

(11) **EP 2 127 617 B1**
(45) Date of publication and mention of the grant of the patent: **26.08.2015**
(21) Application number: 08720935.9
(22) Date of filing: 27.02.2008
(51) Int. Cl.: B05C 5/00, B05C 11/10, B05C 13/02, B05D 1/26, A61F 2/844

(54) **COATING METHOD AND COATING DEVICE**
BESCHICHTUNGSVERFAHREN UND BESCHICHTUNGSVORRICHTUNG
PROCÉDÉ DE REVÊTEMENT ET DISPOSITIF DE REVÊTEMENT

(30) Priority: 20.03.2007 JP 2007072803
(43) Date of publication of application: 02.12.2009
(73) Proprietor: Terumo Kabushiki Kaisha, Tokyo 151-0072 (JP)
(72) Inventor: KINDAICHI, Shori, Fujinomiya-shi Shizuoka 418-0015 (JP); HARADA, Yasukazu, Fujinomiya-shi Shizuoka 418-0015 (JP); TAKEDA, Kazuyuki, Fujinomiya-shi Shizuoka 418-0015 (JP)
(74) Representative: Dossmann, Gérard
(86) International application number: PCT/JP2008/053387
(87) International publication number: WO 2008/114585

(56) References cited:
- WO-A1-01/91918
- WO-A1-01/91918
- WO-A1-03/059410
- WO-A1-2004/012784
- JP-A- 2004 516 935
- JP-A- 2005 514 988
- JP-A- 2005 534 399
- US-A1- 2004 018 296

## Description

### TECHNICAL FIELD

Generally, a stent which is a kind of a medical device is a tube shaped appliance and is used for medical purposes such as maintenance of a state in which a stenosis portion occurring in a blood vessel or other tubular lumen inside a living body is dilated, reinforcement of a lumen and the like.

For example, in case of using a stent for the maintenance of the extension portion after a percutaneous transluminal coronary angioplasty (PTCA), re-stenosis ratio is lower compared with that of the case of only the PTCA, but there is a problem that re-stenosis will be confirmed by a ratio of approximately 20% to 30%. A main cause of the re-stenosis after the stent placement is intimal hyperplasia. Consequently, there has been developed a drug eluting type stent which prevents the re-stenosis by coating a stent with a medicine capable of repressing migration & proliferation of a vascular smooth muscle cell, which are the direct cause of the intimal hyperplasia and by eluting the drug at the stent placement region.

As the medicine, there is used taxol (paclitaxel), mitomycin C, adriamycin, genistein, tyrphostin, cytochalasin, sirolimus (rapamycin) or the like.

When the coating is done, a coating liquid in which this medicine and a biocompatible polymer are dissolved into a solvent is used and applied on a portion of a stent or on the entire thereof such that a predetermined quantity of medicine will exist on the surface of the stent.

As a coating method which has been employed from the past, there are known a dipping method, a spraying method and the like. The dipping method is a method in which a stent is dipped into a coating liquid, pulled up, dried and solidified so as to form a coating layer on the stent.

However, a stent is formed with a groove or a through hole (hereinafter, referred to as space portion) between linear struts, so that it may happen that a web (membrane between struts) or a bridge is generated at a space portion caused by the dipping. In case of using a stent by being extended, there is a fear for such a web or a bridge to exert an influence on the mechanical function of the stent, a portion of the web or the bridge may be destroyed or peeled after applying the stent and if this will flow until a peripheral vessel, there is also a fear that blood flow obstruction will occur, so that it is not preferable.

On the other hand, the spraying method is a method in which a coating liquid is sprayed over the outer circumference surface and/or the inner circumference surface of a stent while rotating the stent and/or moving the spray nozzle, and drying and solidification are applied so as to form a coating layer. However, with respect to the sprayed coating liquid, the amount thereof becomes larger on the disappearing amount side than the amount attached on the stent and a large amount of coating liquid will be wasted. In particular, many of the medicines included in the coating liquid are generally extremely expensive and if wasting the coating liquid, it incurs steep rise of the cost of the stent itself immediately. Also, many of the medicines included in the coating liquid are medicines having toxicity and it is also necessary to strictly carry out the safety management so as not to scatter the medicine by the spray into the environment, so that also the facilities cost thereof will increase.

Consequently, recently, there has been employed a constitution in which a web or a bridge will not be generated at the space portion by relatively moving a spindle (mandrel) holding the stent and the stent after applying a coating liquid on a stent (see summary and the like of Japanese unexamined patent publication No. 2000-51367).

In addition, there also exists a method in which a principle of an electrode position coating is used and electric charge is applied between spray means and the stent so as to heighten the coating efficiency (see summary, FIG. 1 and the like of Japanese unexamined patent publication No. 2003-205037).

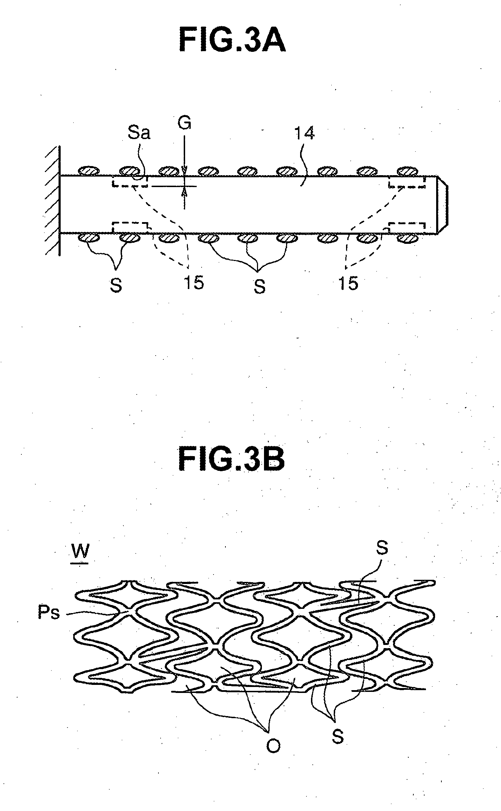

Further, there also exists a method in which a shape pattern of the stent is obtained by a scanning and a coating liquid is applied along this pattern (see summary, FIG. 3 and the like of Japanese PCT unexamined publication No. 2005-514988).

However, with respect to the methods disclosed in the Japanese unexamined patent publication No. 2000-51367 and the Japanese unexamined patent publication No. 2003-205037 mentioned above, either one thereof is not the method in which the coating liquid is applied along the pattern shape of the stent and therefore, it is undeniable that the coating liquid will be wasted.

Also, the method of the Japanese PCT unexamined publication No. 2005-514988 does not have such a defect, but it relates to a method in which the coating liquid is applied by using a solenoid, so that the amount of the medicinal solution to be coated on the stent cannot be set correctly and there is a problem that the effect of reducing the re-stenosis ratio in case of the PTCA mentioned above or the like becomes insufficient.

### DISCLOSURE OF THE INVENTION

The present invention was invented in order to solve the problem mentioned above and has an object to provide a method of applying a coating material (C) to linear struts of a medical device that have spaces formed between said linear struts and to provide a coating layer having a uniform thickness that will be formed extremely accurately.

In particular, the invention relates to a method according to the preamble of claim 1, such as it is for example known from WO 01/91918 A1.

This is achieved by a method of applying a coating material to linear struts of a medical device according to independent claim 1. The dependent claims relate to advantageous embodiments.

When setting an applying route so as to apply aforesaid coating material onto all the surface of aforesaid struts, an effect of the therapeutic substance is brought out at all the regions of aforesaid struts.

With respect to aforesaid strut, when there is set an applying route including at least either one of a section in which repetition application is done and a section in which jumping is done from a certain point to another point, the applying route can be shortened and a speedy application becomes possible. In addition, on an occasion of setting an applying manner, if the section applied overlappingly is reduced or shortened as much as possible and also if an applying route of applying a coating material onto all the surface of the struts is selected, a predetermined quantity of medicine can be applied rapidly and uniformly.

When the applying path at the straight portion of the strut is set at the center position in the width direction of the strut, the coating material is prevented from being disengaged from the strut top and it will never happen further absolutely that a web or a bridge will be generated.

When the applying path at the curved portion of the strut is set at a position deviated by a predetermined length in the width direction from the center position in the width direction of the strut, the coating material is prevented from being disengaged from the strut top depending on the characteristic of the coating material discharged from the nozzle and it will never happen further absolutely that a web or a bridge will be generated.

In particular, if the deviation position of the applying path which is located in the curved portion of aforesaid strut is set from an orbit passing the center of the curved portion of the strut outside, disengagement of the coating material from the strut top is prevented more reliably.

If the setting the applying path is made to be at an intersection point of the center axis lines of the plurality of struts or a vicinity of aforesaid intersection point in the crossing portion of the strut, the coating material is prevented from being disengaged from the strut top even if a plurality of coating layers are formed.

With respect to the applying paths, when the applying paths of a coating layer where applying has already finished and a next coating layer are set identically on the strut, a plurality of coating layers can be formed extremely smoothly and also rapidly.

When the applying path is set so as not to overlap the applying path of the coating layer where applying has already finished for at least a portion thereof, the coating material can be applied with a uniform thickness without being disengaged from the strut top.

When a gap is provided between the outer circumference surface of a mandrel of a holder holding aforesaid medical device and the inner circumference surface of the strut of aforesaid medical device at least at a point initiating the application, not only the application of the coating material but also the detaching of the medical device become extremely smoothly.

If the moving speed of the applicator head is made to be faster during the section in which it passes a plurality of times compared with that during the section in which it passes single time for a predetermined applying route, the coating thickness becomes uniform and also the applying time can be shortened.

If plural kinds of coating materials are applied, medicinal effects become compositive, physical and mental burdens are reduced for a patient and it becomes extremely advantageous.

If the distance between aforesaid nozzle and aforesaid strut is made to be 1µm to 100µm or if the front edge inner diameter of aforesaid nozzle is made to be 5µm to 250µm, the applying can be realized without dropping out the coating material from the strut.

When the applicator head includes a plurality of nozzles and a plurality of dispensers, the application of the coating material can be realized rapidly.

When the coating apparatus includes a second position information obtaining means for measuring position information of Z-direction displacement in the orthogonal coordinate system on the surface of aforesaid strut, the application of the coating material becomes possible corresponding to various struts.

If aforesaid control unit controls the movement of aforesaid applicator head and aforesaid nozzle along the applying path set based on the position information which was obtained by the first position information obtaining means obtaining the position information of the X-direction and the Y-direction in aforesaid orthogonal coordinate system and also, such that the distance between aforesaid applicator head and aforesaid strut set based on the position information of aforesaid Z-direction displacement, which aforesaid second position information obtaining means obtained is to be adjusted, or if aforesaid medical device, aforesaid applicator head and the like are housed in a chamber for which temperature and humidity are controlled, it is possible to form a coating layer having a uniform thickness extremely accurately.

Still further objects, features and characteristics of the present invention will become clear by making allowance for preferable embodiments which will be exemplified in the explanation and the attached drawings hereinafter.

### BRIEF DESCRIPTION OF DRAWINGS

FIG. 1 is a schematic front elevational view of a coating apparatus relating to the present invention;
FIG. 2 is a schematic front elevational view showing a holder and an applicator head;
FIG. 3A is an enlarged cross-section view of a main portion of FIG. 2, and FIG. 3B is a plan view showing a position of a stent corresponding to FIG. 3A;
FIG. 4 is a schematic cross-sectional correspondence view along a 4-4 line in FIG. 1;
FIG. 5 is a cross-section view showing an applying state of a plurality of coating layers;
FIG. 6 is a plan view showing an applying path of a curved portion;
FIG. 7 is a flowchart of a coating method; and
FIG. 8 is a flowchart of the coating method subsequent to FIG. 7.

### BEST MODE FOR CARRYING OUT THE INVENTION

Hereinafter, exemplified embodiments of the present invention will be explained in detail with reference to the drawings.

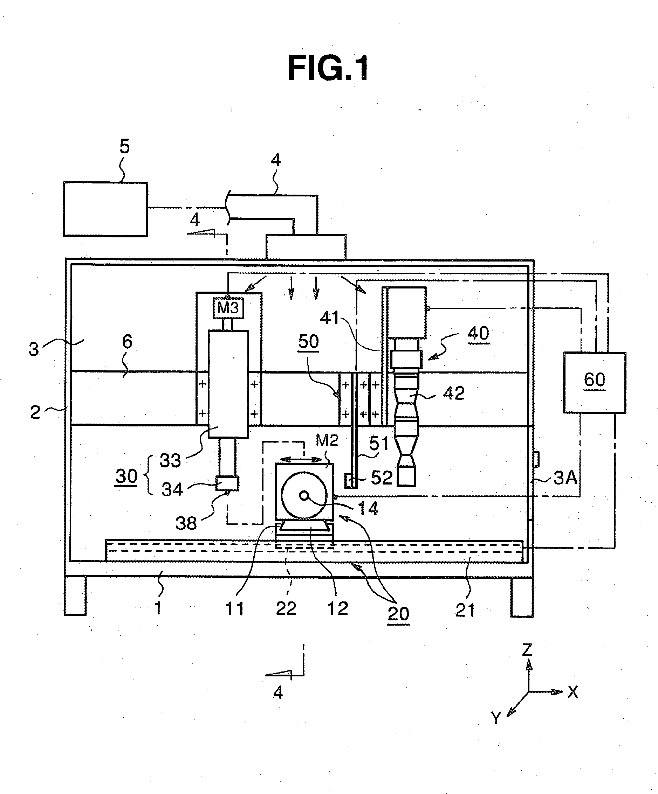

In a coating apparatus of this exemplified embodiment, as shown in FIG. 1, an air-tight chamber 3 is formed in the inside thereof by covering a frame 2 provided elevationally on a base 1 from the outer surface with a transparent synthetic resin plate (not shown). In the chamber 3, a duct 4 is interlinked to a top portion thereof, and air whose temperature and humidity is controlled is supplied from an air conditioner 5, the inside of the chamber is made to be a state with constant temperature and humidity, and drying and solidifying conditions when a coating material C mentioned later is applied on a stent W are always kept constant.

At a lower portion of the inside of the chamber 3 there are provided a holder 10 for holding the stent W which is a medical device and moving means 20 for moving the holder 10, and at an intermediate portion, there are mounted on a support frame 6 laterally bridged to the frame 2 with an applicator head 30 which applies the coating material C on a strut S on the stent W; first position information obtaining means 40 which obtains position information of X-Y-directions in an orthogonal coordinate system on the surface of the stent W, in other words, on the surface of the strut S; and second position information obtaining means 50 which obtains position information of Z-direction in the same orthogonal coordinate system.

On the other hand, at an outside portion of the chamber 3 there is provided a control unit 60 and it is constituted such that the holder 10, the moving means 20, the applicator head 30 and both the position information obtaining means 40, 50 are to be controlled.

However, it is enough if there exist at least the holder 10 and the applicator head 30 in the inside of the chamber 3 and other means may not be always in a state in which the temperature and humidity are controlled.

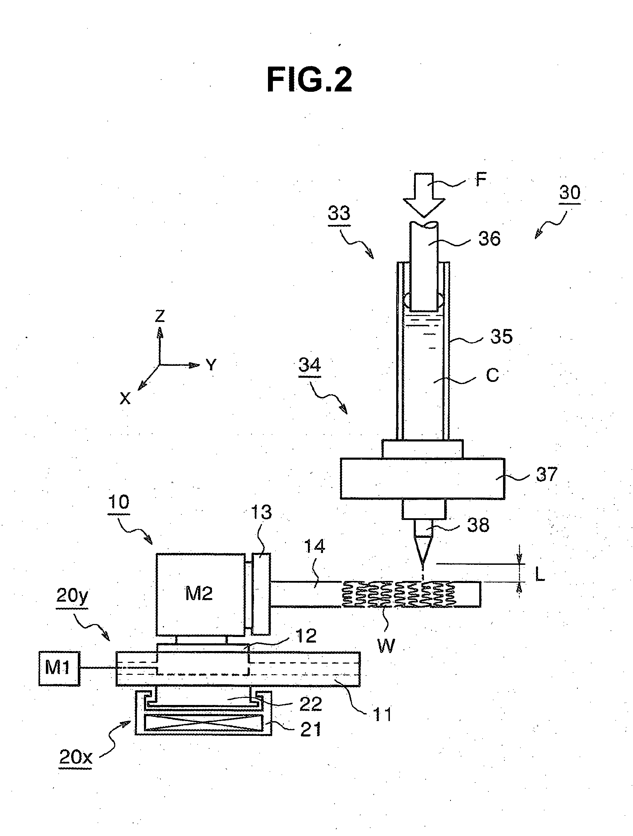

It will be described further in detail. First, the holder 10 is moved in the X-Y-directions by means of the moving means 20. As shown in FIG. 2, first moving means 20x of X-direction is means of, so-called, a linear motor system, and a base plate 11 of the holder 10 is mounted on a movable table 22 which is moved along a traveling rail 21 which is a driving source. A slide portion 12 is placed on the base plate 11 of the holder 10 and at the slide portion 12 there is provided, for example, with second moving means 20y of Y-direction which is composed of a screw-transfer mechanism or the like which is rotated by means of a motor M1. There are provided on the slide portion 12 with a motor M2 and a chuck portion 13, and a proximal end of a mandrel 14 on which the stent W is provided detachably at the outer circumference thereof is chucked at the chuck portion 13 and it is constituted such that it will be rotated rotatably in forward and reverse directions by means of the motor M2.

The outside diameter of the mandrel 14 is approximately the same as or a little bit larger than the inside diameter of the stent W, and the mandrel 14 is exchangeable in conformity with the inside diameter of the stent W and there are prepared mandrels having several kinds of outside diameters in which any one thereof is coated with a black coating material so as to absorb lights and a contrast ratio between the mounted strut S of the stent W and space portions O is to be heighten.

Also, there are formed for the mandrel 14, as shown in FIG. 3A, with concave portions 15 on the outer circumference surface thereof. If the concave portion 15 is formed on the outer circumference surface of the mandrel 14, a gap G occurs between the outer circumference surface of the mandrel 14 and the inner circumference surface Sa of the strut S of the stent W when the stent W is mounted on the mandrel 14. Owing to this gap G, when the coating material C is applied on the strut S and even if the coating material C runs out to the side surface of the strut S, the coating material C can be prevented from turning around a portion between the surface of the mandrel 14 and the inner surface of the stent W and it is possible, without generating a web or a bridge, to form a coating layer having a uniform thickness on the strut S of the stent W. Furthermore, it is possible to avoid also the difficulty of releasing in case of releasing the stent W from the mandrel 14. The turning around of the coating material C occurs easily at a starting point from which the coating material C is applied on the strut S, so that it is allowed for the forming of the concave portion 15 to exist only at a portion corresponding to an application start point shown by "Ps" in FIG. 3B, in other words, only at the under portion of, so-called, an X shape portion (crossing portion of the strut S) which exists in one end region of the strut S. However, it is not limited only by this, it is also allowed to form them widely such that the concave portions 15 exist at the inner circumference surface of all the struts S other than the both end portions, and it is also allowed to form them partially.

Here, the stent W is a stent generally having a cylindrical shape for the whole shape thereof and is composed of bent or curved linear struts S having a predetermined width and space portions O which are formed between the struts S, and the stent is manufactured from a biocompatible and biostable material. For example, as a metal material, there can be cited stainless steel, Ni-Ti alloy, tantalum, titan, gold, platinum, inconel, iridium, tungsten or cobalt alloy (including cobalt-chromium-nickel alloy). As a high-polymer material, for example, there can be cited polytetrafluoroethylene, polyethylene, polypropylene, polyethylene terephthalate or polyamide. As a biodegradable high-polymer material, for example, there can be cited polylactic acid, polyglycolic acid, polylactide, polyglycolide, polyparadioxanone, trimethylene carbonate, ε-caprolactone and the like or a mixture, a copolymer of those above.

Also, the coating material C is a material including at least a solvent, a polymer and a therapeutic substance. It is preferable for a polymer usable as the coating material C to be a material having appropriate adherence with respect to the strut S and having film formability which can follow the deformation of the stent W. It is enough for the polymer to have whichever of biodegradability and non-biodegradability and it is preferable for the polymer to have an excellent characteristic in biocompatibility in order to minimize inflammation of a blood vessel wall. Also, in case of non-biodegradability, it is preferable to select a polymer controllable such that the therapeutic substance will be eluted over time and in case of biodegradability, it is preferable to select a polymer which will be decomposed in an appropriate time period. For example, as a biodegradable polymer, there can be cited polylactic acid, polyglycolic acid, poly-butyric acid, poly-hydroxybutyric acid, polyparadioxanone, trimethylene carbonate, ε-caprolactone, polymalic acid, poly-α-amino acid, collagen, laminin, heparan sulfate, fibronectin, vitronectin, chondroitin sulfate, hyaluronic acid and the like or a mixture, a copolymer of those above.

As a non-biodegradable polymer, there can be cited silicone, cellulose-based polymer, polyurethane, polyester, polymethacrylate, polyethylene-oxide, polyvinyl alcohol, polyethylene glycol, polyvinyl pyrrolidone, poly-acrylic acid or the like.

For the therapeutic substance, as an example of a medicine which suppresses migration & proliferation of vascular smooth muscle cells, there can be cited taxol (paclitaxel), actinomycin C, mitomycin C, adriamycin, genistein, tyrphostin, cytochalasin, sirolimus (rapamycin), tacrolimus, everolimus or the like.

It is preferable for the solvent to be a solvent which dissolves a polymer, a therapeutic substance and the like, but it is acceptable to select a solvent which can disperse those uniformly. Also, it is preferable for the stent W to be provided with wettability and an appropriate vaporization speed and the stent is selected by achieving a balance therebetween. As a preferable solvent, there can be cited acetone, N-methylpyrrolidone, dimethyl sulfoxide, toluene, xylen, methylene chloride, chloroform, fleon, dioxane, acetic ether, tetrahydrofuran, dimethylformamide, dimethylacetamide or a mixture of those above.

Further, it is also allowed to add additive agents aiming for adjustment of the physical characteristic of the polymer film, improvement of adherence with respect to the stent W, viscosity adjustment of the coating solution, oxidation inhibition of the therapeutic substance or the like. As those additives, for example, there can be cited glycerol, triacetyl glycerine, ethylene glycol, triethylene glycol, polyethylene glycol, polypropylene glycol, propylene glycol, polyalkylene-oxide, sebacic acid ester, citric acid ester, phthalic acid ester or the like.

The coating material C discharged from a nozzle 38 mentioned later is deployed on the strut S, so that the viscosity of the coating material C is selected to be 0.1cp to 10cp and preferably to be 1.0cp to 4.0cp. When the viscosity is higher than this level, there exists a case in which a large pressure is necessary for the discharge or the coating material can not be discarged from the thin nozzle 38 and when the viscosity is lower than this level, the discharged coating material C runs out of the strut S and there may happen a case in which a uniform coating layer can not be formed.

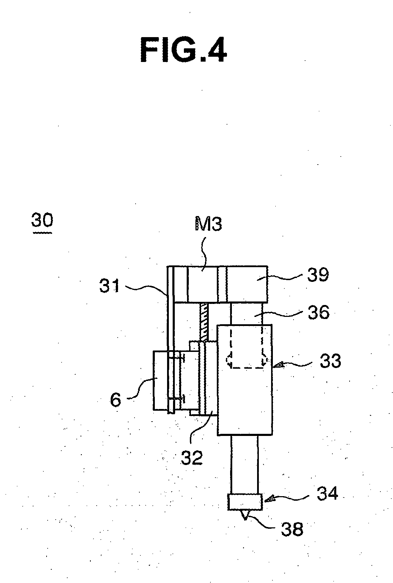

The applicator head 30 includes, as shown in FIG. 4, a dispenser 33 composed of, for example, a syringe operation mechanism and a nozzle portion 34 for discharging the coating material C, which are mounted on a bracket 31 mounted on the support frame 6 through, for example, a vertical table 32 being moved in the Z-direction by means of the screw-transfer mechanism or the like driven by a motor M3 and which gradually discharge the coating material C retained in the inside thereof.

The dispenser 33 of this exemplified embodiment includes, as shown in FIG. 2, a cylinder portion 35 in which the coating material C is retained inside, a piston portion 36 provided in the inside of the cylinder portion 35 slidably, and a driving unit 39 of a motor, a liquid pressure mechanism or the like which presses the piston portion 36 by a predetermined force F.

The nozzle portion 34 is constituted by an mounting member 37 which is provided at the lower end of the cylinder portion 35 and the nozzle 38 which is hung down from the mounting member 37, and there is formed a flow route (not shown) on which the coating material C flows from the cylinder portion 35 to the nozzle 38.

The outer diameter of the tip of the nozzle 38 is 10µm to 1000µm, and the inner diameter of the tip of the nozzle is selected to be 1µm to 500µm and preferably to be 5µm to 250µm such that the coating material C having the viscosity mentioned above is to be pushed out with a predetermined discharge speed. In case of 5µm or less, the coating material C does not flow out smoothly and also, a large pressure will be required for the discharge, and in case of 250µm or more, there is a fear that the coating material C can not be applied substantially smoothly on the top of the stent W which is used presently.

Also, it is preferable for the nozzle 38 to be polished such that irregularity of the surface thereof will be made less as much as possible in order to avoid the attachment of the discharged coating material C. Consequently, it is preferable to use, for example, stainless steel, carbon steel, nickel, chromium, glass, aluminum oxide, zirconium oxide, diamond, a mixture of those above or the like, which is a material easily being processed finely.

In particular, the dispenser 33 of this exemplified embodiment is, as shown in FIG. 2, constituted such that it is to be deposited on the surface of the strut S being apart therefrom in a way that the distance L between the nozzle 38 and the strut S will become a predetermined distance and the coating material C will be pushed out continuously without a break from the nozzle 38 directed to the surface of strut S. If there is employed such a continuous push-out system, uniform application of the coating material C becomes possible with respect to the whole surface of the stent W and also, the continuous push-out system can quantitatively discharge, so that controllability is excellent and quantitative adjustment of the therapeutic substance can be carried out accurately reliably. Furthermore, in the inside of the chamber which is adjusted for the constant-temperature & constant-humidity, the condition by which the coating material C is dried and solidified is constant, so that production of the stent W on which the coating material C is deposited can be carried out easily and also rapidly.

As the distance L between the nozzle 38 and the strut S, it is selected to be 0.1µm to 200µm and preferably to be 1µm to 100µm. When the distance L is too much larger than this distance, there is a problem in which the coating material C will be interrupted and when it is too much narrower, there is a problem in which the coating material C runs out of the surface of the strut.

It should be noted in this exemplified embodiment that the applicator head 30 is a head having one piece of nozzle 38 and one piece of dispenser 33, but it is also allowed to employ a head having a plurality of nozzles 38 and a plurality of dispensers 33. In a case in which the head has a plurality of nozzles 38 and a plurality of dispensers 33 in this manner, it is possible to carry out the application of the coating material C in shorter time, productivity is improved and it becomes advantageous also for the production cost.

The first position information obtaining means 40 is, as shown in FIG. 1, constituted by imaging means which is mounted position-fixedly on a bracket 41 which is mounted on the support frame 6. More specifically, the first position information obtaining means 40 is means including a camera unit 42 and a line sensor unit (not shown) which is arranged so as to be extending in an axis direction of the stent W, scans the surface of the stent W in synchronization with the rotation of the stent W of the holder 10, obtains an image of the surface of the stent W and communicates it to the control unit 60. As a start position at which the line sensor unit obtains the surface image of the stent W, any position is acceptable, but preferably it is set on, for example, an axis line corresponding to the application start point Ps mentioned above. It should be noted that the camera unit 42 and the line sensor unit belong to matters publicly known, so that the details thereof will be avoided.

As mentioned above, it is constituted such that the mandrel 14 is coated with a black coating material to absorb light and to heighten a contrast ratio between the strut S of the mounted stent W and the space portion O and the light will be absorbed, so that with respect to the image of the stent surface, the luminance of the strut S is high and the luminance of the space portion O is low. Consequently, it is possible for the control unit 60 to distinguish the strut S and the space portion O and to output the coordinates of the strut S, that is, to output the position information of the X-Y-directions of the strut S by binarizing the obtained surface image of the stent W depending on appropriate luminance. Further, the control unit 60 calculates the coordinates of an orbit passing the center of the strut S based on the obtained position information of the X-Y-directions (X-Y coordinates of the strut S), and the obtained data of the center orbit are stored in the memory of the control unit 60. In case of applying the coating material C, it is essential that the coating material C is prevent from being disengaged from the strut S and also for this reason, it is extremely important that the center of the strut S is to be specified.

The second position information obtaining means 50 is, as shown in FIG. 1, constituted by displacement measuring means of the Z-direction, which is mounted position-fixedly on the lower end of a bracket 51 mounted on the support frame 6 and more specifically, it is constituted by a laser displacement sensor 52 which is called a so-called vertical sensor and measures displacement of the Z-direction of the strut S.

The strut S is a strut whose surface is not smooth in a precise sense and has irregularity, and in order to apply the coating material C quantitatively precisely on the strut having such irregularity, the distal end of the nozzle 38 must be moved so as to become in parallel with the surface of the strut S precisely and a predetermined amount of coating material C must be applied. Consequently, in this exemplified embodiment, by using the laser displacement sensor 52, the obtaining of the position information of the strut S is started from the predetermined position of the stent W, for example, from the application start point Ps, the laser displacement censor 52 is scanned along the orbit passing the center of the strut S rotating the stent W in the forward and reverse directions, and the displacement data of the Z-direction of the whole stent W are collected and obtained. The obtained displacement data are communicated to the control unit 60 and stored in the memory.

It should be noted in this exemplified embodiment that there is provided as the second position information obtaining means 50 with one piece of the laser displacement sensor 52, but when a plurality of the laser displacement sensors 52 are provided, the obtaining of the position information can be realized more rapidly.

The control unit 60 includes a processor, a monitor, a keyboard and the like although they are not shown in the drawing and based on aforesaid various kinds of position information, it determines setting of an applying manner by which the coating material is applied and an applying path along which the applicator head 30 applies the coating material C on the strut S of the stent W. Also, it controls the rotation of the mandrel 14 in the holder 10, the movement of the moving means 20, the discharge amount of the coating material C discharged from the applicator head 30, the scanning of the imaging means and of the vertical sensor and the like.

Here, an applying manner means an applying route in a case in which the application is executed along the strut S of the stent W. It is preferable for the applying route to be a route which does not have a section in which the repetition application is done and which can apply all the surface of the strut S continuously, but there is a case for the stent W having struts S intersected complicatedly in which it is difficult to set a route having no section in which the repetition application is done. In such a case, there may be provided a section in which the repetition application is done or there may be provided a section in which jumping is done from a certain point on the strut S to another point thereof. In this manner, the applying route can be shortened by providing for a portion with a section of repetition application or a section of jumping. Also, in a section of repetition application (section of passing a plurality of times), the medicine can be applied on all the surface of the stent W uniformly by setting the speed to be faster than the moving speed during the section of passing single time, and it is possible to sufficiently exert an effect of reducing a re-stenosis ratio in case of PTCA or the like.

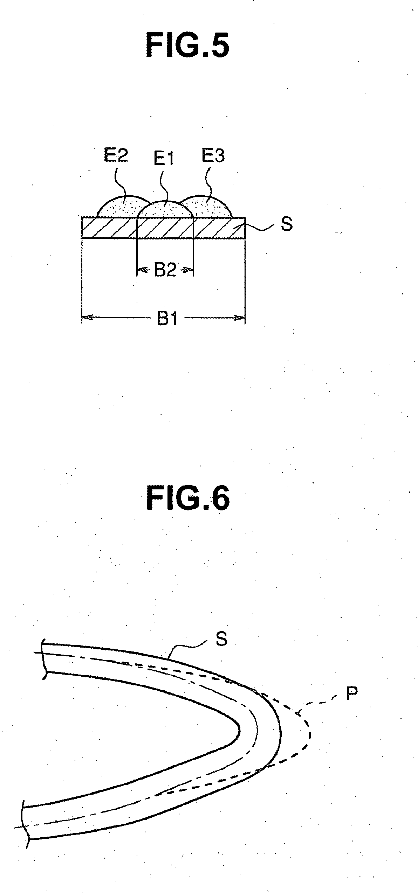

Also, on an occasion of the determination of the applying path, it is preferable to implement various kinds of countermeasures described hereinafter.
(1) In case of applying the coating material C on the strut S of the stent W, it is preferable, at a straight portion of the strut S, to set the orbit passing the center of the strut S to be the applying path. However, at a curved portion of the strut S, it is preferable to set the orbit passing the position deviated by a predetermined length in the width direction of the strut S be the applying path. The coating material C is a liquid and exists on the surface of the strut S in a state of being raised by surface tension, and even if this is dried and solidified, a coating layer having an arc shaped cross section will be formed. Accordingly, when the application is executed a plurality of times, it easily becomes in a state of being raised in an arc shape and it is not preferable. Consequently, as shown in FIG. 5, it is preferable for a coating layer E2 at the second time to be formed as an applying path of the orbit that passes a position deviated by a predetermined length from a coating layer E1 at the first time of the strut S and it is preferable for a coating layer E3 at the third time to employ an orbit passing a position different from those of the applying paths of the first and second times as the applying path thereof. It should be noted that it is preferable for this deviated length to take a width B1 of the strut S and a width B2 of the coating layer E into consideration.
(2) On an occasion of the determination of the applying path, it is preferable for the applying paths to be made different in connection with the linear shaped strut portion and the curved strut portion. At the linear shaped strut portion, it is enough basically if an orbit passing the center of the width direction of the strut S is made to be the applying path. However, at the curved strut portion, if an orbit passing the center of the width direction of the strut S is made to be the applying path, the coating material C discharged from the applicator head 30 is affected by the viscosity thereof, the falling speed from the nozzle or the like and it does not follow along the moving orbit of the applicator head 30. Accordingly, in this exemplified embodiment, as shown in FIG. 6, a curved line P (dotted line) passing the outside of a curved line (dashed-dotted line) which passes the center of the curved portion of the strut S is set as the applying path.

Here, the applying path P of the dotted line is sticking out to an outside portion of the strut S, and this is based on a knowledge, when the coating material C applied on the strut S by being pushed out continuously, that the coating material C can be prevented more reliably from being disengaged from the strut S depending on the characteristic of the coating material C.
(3) At the crossing portion of the strut S, it is preferable for an intersection point of a center axis line of aforesaid plurality of struts S or the vicinity of the intersection point thereof to be made as the applying path. At the portion at which the strut S is intersected, the applying path P will also be intersected, so that if intersection point of a center axis line of the struts S or the vicinity of the intersection point thereof is set to be the applying path, it is possible to prevent running off the coating material C and it becomes preferable.

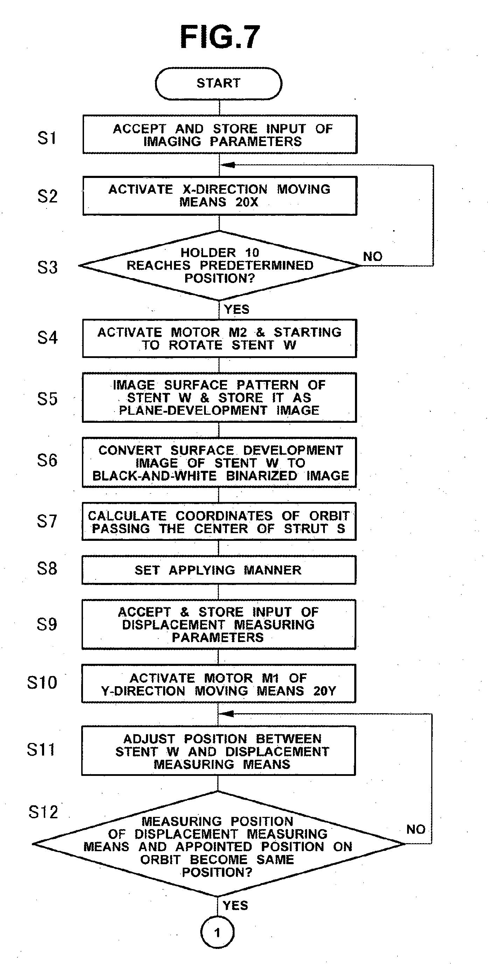

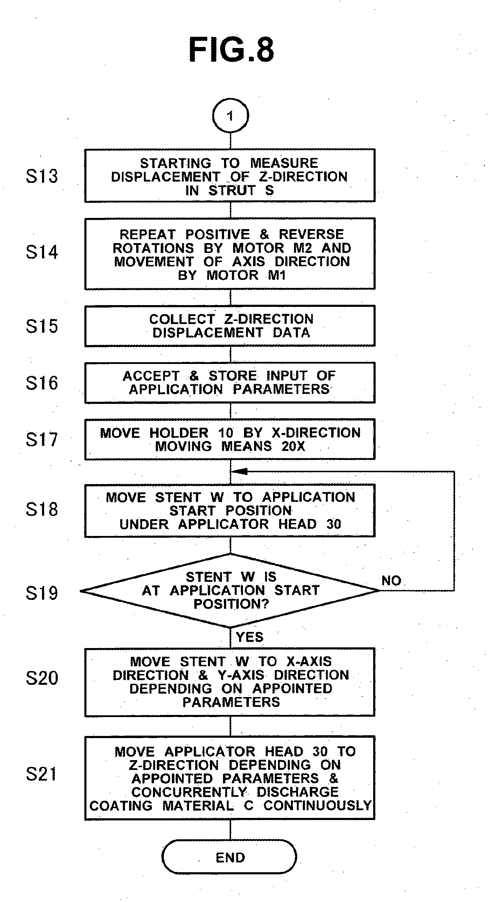

Next, a coating method will be explained. FIG. 7 and FIG. 8 are flowcharts showing a coating method.

### <Preparation Process>

First, on an occasion of starting the coating, the air conditioner 5 is activated and the inside of the chamber 3 is made to be a condition of constant temperature and constant humidity. Then, at the support frame 6, there is set the syringe operation mechanism 33 formed by combining the nozzle portion 34 including the nozzle 38 having an inside diameter corresponding to the width of B1 of the strut S and the coating material C, the cylinder portion 35 retain the coating material C and the like.

On the other hand, the stent W is attached on the mandrel 14 and thereafter, is mounted on the chuck portion 13 of the holder 10 which is positioned at a standby position. At that time, the application start point Ps of the strut S is to be set so as to position over the concave portion 15 of the mandrel 14. Here, in case of having the moving means 20 at the lower portion of the chamber 3, as shown in FIG. 1, the standby position is in the vicinity of an entrance portion 3A of the chamber 3.

### <Imaging Process>

The control unit 60 accepts an input of imaging parameters and the inputted imaging parameters are stored in a storage device (S1). The imaging parameters are, for example, inputted from a keyboard relying on an operator. The imaging parameters include a rotation speed of the mandrel 14, the number of imaging lines, an imaging line width and an operation speed when imaging.

The control unit 60 instructs a start of imaging after storing the inputted imaging parameters. Concurrently, the X-direction moving means 20x is activated (S2). When the X-direction moving means 20x is activated, the holder 10 moves from the standby position under the first position information obtaining means 40 by means of the traveling rail 21. The control unit 60 confirms that the holder 10 reached the predetermined position (S3) and if the holder 10 reached the predetermined position, it activates the motor M2 of the holder 10 and starts the rotation of the stent W (S4).

On the other hand, the line sensor of the first position information obtaining means 40 starts the imaging by the instruction of the start of imaging, scans the surface of the stent W relatively, because the stent W rotates and images the surface pattern of the stent W (S5). The imaged picture is stored in the storage device (for example, memory, hard disk or the like) of the control unit 60 as a plan view development image. In addition, it is also allowed to employ a constitution in which confirmation is possible by watching by outputting it to a monitor.

With respect to the surface image of the stent S, the luminance of the strut S is high and the luminance of the space portion O is low, so that the control unit 60 converts it to a black-and-white binarized image by setting a certain luminance as a boundary (S6) and calculates the coordinates of the orbit passing the center of the strut S by a thinning processing of the width of the strut S (S7).

### <Setting Process of Applying Manner>

By judging from the surface image of the stent W obtained by aforesaid process and by taking into account the necessity/unnecessity and the position of the section in which the repetition application is done and the section in which the jumping is done, all the surfaces of the strut S are applied, and the applying route is set such that the section in which the repetition application is done and the section in which the jumping is done will be reduced or shortened as much as possible (S8).

### <Displacement Measurement Process of Z-Direction>

Next, the control unit 60 accepts an input of displacement measuring parameters of the second position information obtaining means 50.which is the displacement measuring means of the Z-direction and stores it (S9). These displacement measuring parameters are also inputted by an operator. The displacement measuring parameters include data of the measuring start position, the measuring direction, the direction at the branch point, the measuring speed and the interval of measurement point.

The control unit 60 activates the motor M1 of the Y-direction moving means 20y after storing the displacement measuring parameters (S10). At that time, if it is necessary, while observing by a video camera and a monitor, the stent W and the measuring position of the displacement measuring means are adjusted such that the measuring position of aforesaid displacement measuring means and the specified position on the orbit become the same position (S11).

When the measuring position and the specified position become the same position by this adjustment (here, a fact that these coincided is inputted by an operator to the control unit 60 (S12: Yes)), the control unit 60 instructs the second position information obtaining means 50 on the start of the measurement of the Z-direction displacement in the strut S (S13). Concurrently, the control unit 60 makes positive and reverse rotations by the motor M2 and the movement of the axis direction by the motor M1 repeat. Thus, the stent W repeats the rotation and the movement of the axis direction (S14).

Thus, the second position information obtaining means 50 moves along the orbit passing the center of the strut S, so that the control unit 60 collects the displacement data of the Z-direction (S15). These displacement data are stored in the storage device of the control unit 60 together with the coordinates of aforesaid center orbit.

### <Applying Process>

The control unit 60 accepts an input of application parameters, and stores this (S16). The application parameters are also inputs by an operator. The application parameters include data of the application start position, the application direction, the direction at the intersection, setting of the orbit adjustment section, the deviated length of the orbit, the applying speed, the discharge speed of the coating material C, the height of the applicator head, the number of applications (number of layers) and the selection of the applicator heads.

The control unit 60 instructs the application start after storing the application parameters. Concurrently, the control unit 60 instructs the movement of the holder 10 by the X-direction moving means 20x (S17). Thus, the stent W is moved to the application start position under the applicator head 30 (S18). If the stent W reached the application start position under the applicator head 30 (S19: Yes), there are instructed the positive and reverse rotations by the motor M2 and the movement of the axis direction by the motor M1 and the stent W is moved in the X-axis direction and in the Y-axis direction depending on the appointed parameter according to the positive and reverse rotations by the motor M2 and according to the movement of the axis direction by means of the motor M1 (S20). Concurrently, the applicator head 2 is moved in the Z-axis direction by the motor M3 depending on the appointed parameters (S21). At that time, the coating material C is discharged from the applicator head 2 continuously. Thus, the applicator head 2 applies the coating material C moving along the predetermined applying path.

During the application, the space between the distal end of the nozzle 38 and the stent W is in a state of being filled by the coating material C, so that the coating material C is discharged by a fixed amount and the amount of the therapeutic substance on the stent W is also set to be a predetermined value reliably. Furthermore, the web and the bridge will be never generated between the struts S and the coating layer E can be formed extremely accurately.

When the coating is completed, the holder 10 is moved to the standby position by the X-direction moving means 20x, the mandrel 14 is removed from the holder 10 and is took out to the outside of the chamber 3, and the stent W is released from the mandrel 14.

The present invention is not limited only by the exemplified embodiments mentioned above and various modifications are possible in the technical idea of the present invention depending on a person skilled in the art. For example, the exemplified embodiments mentioned above relate to a case in which one kind of the coating material C is applied, but depending on situation, it is also allowed to use plural kinds of coating materials. In case of applying a plurality of coating materials C, there are provided a plurality of syringes, nozzles and syringe operation mechanisms and the coating is executed by changing-over them sequentially. The sequential changeover of these syringes operation mechanisms and the like is controlled by the control unit 60. Here, the different coating material C means a case in which the polymer is different or the polymer is the same and the amount thereof is different; the therapeutic substance is different or the amount of the therapeutic substance is different; or the solvent is different. In this manner, if plural kinds of coating materials are used, the medicinal effects become compositive and it becomes extremely advantageous in cases such as a case in which physical and mental burdens for a patient are reduced or the like.

Also, in this exemplified embodiment, the holder 10 is moved by the first and second moving means 20x, 20y and the applicator head 30 and the first and second position information obtaining means 40, 50 are fixed, but there may not always employed a constitution in which only the holder 10 is always moved and other parts must be fixed. It is allowed for these movements to be relative and thus, it is enough if predetermined moving means is installed suitably.

Further, also with respect to the movement directions of the holder 10, the applicator head 30 and the first and second position information obtaining means 40, 50, there may not always employed a constitution in which the applicator head 30 and the holder 10 are moved relatively to two directions orthogonal each other in the horizontal surface or in which the movement is executed in the vertical direction relatively, and it is also possible to select the most suitable condition depending on the shape or the like of the stent W.

### <Inventive Example 1>

For the stent W, there was used the following stent and the coating is executed.
Stent W: Inside Diameter of 1.7mm, Thickness of 150µm, Full Length of 30mm
Width of Strut S (minimum): 107µm
Radius of Curvature of Strut S (minimum): R = 0.1mm

The pattern of strut S is a pattern obtained by connecting rings composed of waveforms with linear shaped links.

The material of the stent W is stainless steel.
Viscosity of Coating Material C: 2.5cp (20°C)
Discharge Speed of Coating Material C: 0.0067µl/sec
Adjustment Deviated Length of Orbit of Curved Portion
   : 40µm in Y-direction (Stent Circumferential Direction)
   : 60µm in X-direction (Stent Long Axis Direction)
Applying Speed on Orbit Passing the Center: 2.4mm/sec
Applying Speed on Orbit Deviated from the Center: 3.3mm/sec
Applying Speed in Section of Repetition Application: 4.8mm/sec
Inner Diameter of Tip of Nozzle 38: 43µm
Outer Diameter of Tip of Nozzle 38: 200µm
Distance (L) between Stent W and Nozzle 38: 40µm
Number of Applications (Number of Layers): 10 Layers

With respect to the applying manner, there was provided a section in which the repetition application is done at a portion of the applying route, but there was no section in which the jumping is done.

The average thickness of the coating layer of the stent of the inventive example 1 was 40µm and the coating could be realized without the web, the bridge and running out to the stent side surface.

### <Inventive Example 2>

Next, with respect to the stent W, the following stent was used and the coating was carried out.
Stent W: Inside Diameter of 1.7mm, Thickness of 150µm, Full length of 30mm
Width of Strut S (minimum): 104µm
Radius of Curvature of Strut S (minimum) R = 0.07mm

The pattern of the strut S is obtained by connecting rings composed of waveforms with linear shaped links.

The material of the stent W is stainless steel.
Viscosity of Coating Material C: 2.5cp (20°C)
Discharge Speed of Coating Material C: 0.0056µl/sec
Adjustment Deviated Length of Orbit of Curved Portion
   : 32µm in Y-direction (Stent Circumferential Direction)
   : 52µm in X-direction (Stent Long Axis Direction)
Applying Speed on Orbit Passing the Center: 4.4mm/sec
Applying Speed on Orbit Deviated from the Center: 5.7mm/sec
Applying Speed in Section of Repetition Application: 8.8mm/sec
Inner Diameter of Tip of Nozzle 38: 43µm
Outer Diameter of Tip of Nozzle 38: 200µm
Distance (L) between Stent W and Nozzle 38: 40µm
Number of Applications (Number of layers): 10 layers

With respect to the applying manner, there was provided a section in which the repetition application is done at a portion of the applying route, but there was no section in which the jumping is done.

The average thickness of the coating layer of the stent of the inventive example 2 was 40µm and the coating could be realized without the web, the bridge and running out to the stent side surface.

### INDUSTRIAL APPLICABILITY

In the present invention, it becomes possible to reduce the re-stenosis ratio of the dilation portion drastically after the percutaneous transluminal coronary angioplasty (PTCA).

Further, the present application is based on the Japanese Patent Application No. 2007-72803 filed on March 20, 2007 and the disclosed contents thereof are referred to and incorporated as a whole.

## Claims

1. A method of applying a coating material (C) to linear struts (8) of a medical device that have spaces formed between said linear struts (S) extending continuously, a coating method **characterized by** comprising:
a process for optically scanning the surface of said medical device and for obtaining position information of said struts (S);
a process for calculating a predetermined position in the width direction of said strut from said position information;
a process for setting an applying manner to apply said coating material (C) based on said predetermined position;
a process for setting an applying path (P) accommodating to said applying manner; and
a process for moving relatively between said medical device and an applicator head (30). along said applying path (P) and for applying said coating material (C) at least once, wherein
said coating material (C) is discharged continuously from said applicator head (30) to the surface of said struts (8).
**characterized in that** said process for setting the applying path (P) sets the path at a position deviated by a predetermined length in the width direction from the center position in the width direction of said strut (S) in a curved portion of said strut (S).

2. The coating method according to claim 1, **characterized in that** said process for setting the applying manner sets an applying route so as to apply said coating material (C) onto all the surface of said strut (S).

3. The coating method according to claim 1 or 2, **characterized in that** said process for setting the applying manner sets, with respect to said strut (8), an applying route including at least either one of a section in which repetition application is done and a section in which jumping is done from a certain point to another point.

4. The coating method according to any one of claims 1 to 3, **characterized in that** said process for setting the applying path (P) sets the path at the center position in the width direction of said strut (8) in a straight portion of said strut (S).

5. The coating method according to claim 1, **characterized in that** the deviation position of the applying path (P) which is located in the curved portion of said strut is set from an orbit
passing the center of the curved portion of said strut (8) outside.

6. The coating method according to any one of claims 1 to 5, **characterized in that** said process for setting the applying path (P) sets the applying path at an intersection point of the center axis lines of said plurality of struts (8) or a vicinity of said intersection point in the crossing portion of said strut (S).

7. The coating method according to any one of claims 1 to 6, **characterized in that** said process for setting the applying path (P) identically sets the applying paths (P) of a coating layer (E1) where applying has already finished and a next coating layer (E2) on said strut (S).

8. The coating method according to any one of claims 1 to 7, **characterized in that** said process for setting the applying path (P) sets the applying path of the next coating layer (E2) so as not to overlap the applying path (P) of the coating layer (E1) where applying has already finished on said strut (S) for at least a portion thereof.

9. The coating method according to any one of claims 1 to 8, **characterized in that** said process for applying makes a gap
(G) exist between the outer circumference surface of a mandrel (14) of a holder (10) holding said medical device and the inner circumference surface of the strut (S) of said medical device at least at a point initiating the application.

10. The coating method according to any one of claims 1 to 9, **characterized in that** said process for applying makes the moving speed faster during the section in which said applicator head (30) passes a plurality of times compared with the moving speed during the section in which said applicator head (30) passes single time.

## Patentansprüche

1. Verfahren zum Auftragen eines Beschichtungsmaterials (C) auf lineare Streben (8) einer medizinischen Vorrichtung, die zwischen den linearen Streben (S) geformte Räume hat, die sich durchgehend erstrecken, ein Beschichtungsverfahren, das **dadurch gekennzeichnet ist, dass** es Folgendes umfasst:
einen Vorgang zum optischen Abtasten der Oberfläche der medizinischen Vorrichtung und zum Erfassen von Positionsinformationen der Streben (S),
einen Vorgang zum Berechnen einer vorbestimmten Position in der Breitenrichtung der Strebe aus den Positionsinformationen,
einen Vorgang zum Festsetzen einer Auftragsweise, um das Beschichtungsmaterial (C) aufzutragen, auf der Grundlage der vorbestimmten Position,
einen Vorgang zum Festsetzen einer Auftragsbahn (P), die sich an die Auftragsweise anpasst, und
einen Vorgang zum relativen Bewegen zwischen der medizinischen Vorrichtung und einem Auftragskopf (30) entlang der Auftragsbahn (P) und zum Auftragen des Beschichtungsmaterials (C) wenigstens einmal, wobei
das Beschichtungsmaterial (C) durchgehend von dem Auftragskopf (30) zu der Oberfläche der Streben (8) abgegeben wird,
**dadurch gekennzeichnet, dass** der Vorgang zum Festsetzen der Auftragsbahn (P) die Bahn in einem gekrümmten Abschnitt der Strebe (S) an einer Position festsetzt, die um eine vorbestimmte Länge in der Breitenrichtung von der Mittelposition in der Breitenrichtung der Strebe (S) abweicht.

2. Beschichtungsverfahren nach Anspruch 1, **dadurch gekennzeichnet, dass** der Vorgang zum Festsetzen der Auftragsweise einen Auftragsweg so festsetzt, dass das Beschichtungsmaterial (C) auf die gesamte Oberfläche der Strebe (S) aufgetragen wird.

3. Beschichtungsverfahren nach Anspruch 1 oder 2, **dadurch gekennzeichnet, dass** der Vorgang zum Festsetzen der Auftragsweise, Strebe (8), einen Auftragsweg festsetzt, der wenigstens eines von einer Sektion, in der ein Wiederholungsauftrag vorgenommen wird, und einer Sektion, in der ein Springen von einem bestimmten Punkt zu einem anderen Punkt vorgenommen wird, einschließt.

4. Beschichtungsverfahren nach einem der Ansprüche 1 bis 3, **dadurch gekennzeichnet, dass** der Vorgang zum Festsetzen der Auftragsbahn (P) in einem geraden Abschnitt der Strebe (8) die Bahn an der Mittelposition in der Breitenrichtung der Strebe (S) festsetzt.

5. Beschichtungsverfahren nach Anspruch 1, **dadurch gekennzeichnet, dass** die Abweichungsposition der Auftragsbahn (P), in dem gekrümmten Abschnitt der Strebe angeordnet ist, von einer Umlaufbahn, die durch die Mitte des gekrümmten Abschnitts der Strebe (8) hindurchgeht, außerhalb festgesetzt wird.

6. Beschichtungsverfahren nach einem der Ansprüche 1 bis 5, **dadurch gekennzeichnet, dass** der Vorgang zum Festsetzen der Auftragsbahn (P) die Auftragsbahn an einem Schnittpunkt der Mittelachslinien der mehreren Streben (8) oder einer Nachbarschaft des Schnittpunkts in dem Kreuzungsabschnitt der Strebe (S) festsetzt.

7. Beschichtungsverfahren nach einem der Ansprüche 1 bis 6, **dadurch gekennzeichnet, dass** der Vorgang zum Festsetzen der Auftragsbahn (P) die Auftragsbahnen (P) einer Beschichtungslage (E1), wo das Auftragen auf der Strebe (S) bereits geendet hat, und einer nächsten Beschichtungslage (E2) identisch festsetzt.

8. Beschichtungsverfahren nach einem der Ansprüche 1 bis 7, **dadurch gekennzeichnet, dass** der Vorgang zum Festsetzen der Auftragsbahn (P) die Auftragsbahn der nächsten Beschichtungslage (E2) für wenigstens einen Abschnitt derselben so festsetzt, dass sie die Auftragsbahn (P) der Beschichtungslage (E1), wo das Auftragen auf der Strebe (S) bereits geendet hat, nicht überlappt.

9. Beschichtungsverfahren nach einem der Ansprüche 1 bis 8, **dadurch gekennzeichnet, dass** der Vorgang zum Auftragen veranlasst, dass es wenigstens an einem Punkt, der den Auftrag einleitet, einen Spalt (G) zwischen der Außenumfangsfläche eines Dorns (14) einer Halterung (10), welche die medizinische Vorrichtung hält, und der Innenumfangsfläche der Strebe (S) der medizinischen Vorrichtung gibt.

10. Beschichtungsverfahren nach einem der Ansprüche 1 bis 9, **dadurch gekennzeichnet, dass** der Vorgang zum Auftragen die Bewegungsgeschwindigkeit während der Sektion, in welcher der Auftragskopf (30) mehrere Male passiert, schneller macht, verglichen mit der Bewegungsgeschwindigkeit während der Sektion, in welcher der Auftragskopf (30) ein einziges Mal passiert.

## Revendications

1. Procédé d'application d'un matériau de revêtement (C) sur des haubans linéaires (8) d'un dispositif médical qui ont des espaces formés entre lesdits haubans linéaires (S) s'étendant de façon continue, le procédé d'enduction étant **caractérisé en ce qu'**il comprend :
un processus pour balayer optiquement la surface dudit dispositif médical et pour obtenir une information de position desdits haubans (S) ;
un processus pour calculer une position prédéterminée dans la direction de la largeur dudit hauban à partir de ladite information de position ;
un processus pour établir une manière d'application pour appliquer ledit matériau de revêtement (C) d'après ladite position prédéterminée ;
un processus pour établir un chemin d'application (P) qui s'adapte à ladite manière d'application ; et
un processus pour se déplacer relativement entre ledit dispositif médical et une tête d'application (30) le long dudit chemin d'application (P) et pour appliquer ledit matériau de revêtement (C) au moins une fois, dans lequel
ledit matériau de revêtement (C) est déchargé en continu depuis ladite tête d'application (30) sur la surface desdits haubans (8),
**caractérisé en ce que** ledit processus pour établir le chemin d'application (P) établit le chemin en une position écartée d'une longueur prédéterminée dans la direction de la largeur depuis la position centrale dans la direction de la largeur dudit hauban (S) dans une partie courbée dudit hauban (S).

2. Procédé d'enduction selon la revendication 1, **caractérisé en ce que** ledit processus pour établir la manière d'application établit un chemin d'application afin d'appliquer ledit matériau de revêtement (C) sur toute la surface dudit hauban (S).

3. Procédé d'enduction selon la revendication 1 ou 2, **caractérisé en ce que** ledit processus pour établir la manière d'application établit, par rapport audit hauban (8), un chemin d'application qui comprend au moins l'une ou l'autre d'une section dans laquelle une application répétée est effectuée et une section dans laquelle un saut est réalisé d'un certain point à un autre point.

4. Procédé d'enduction selon l'une quelconque des revendications 1 à 3, **caractérisé en ce que** ledit processus pour établir le chemin d'application (P) établit le chemin à la position centrale dans la direction de la largeur dudit hauban (8) dans une partie droite dudit hauban (S).

5. Procédé d'enduction selon la revendication 1, **caractérisé en ce que** la position de déviation du chemin d'application (P) qui est situé dans la partie courbée dudit hauban est établie à partir d'une orbite qui fait passer le centre de la partie courbée dudit hauban (8) à l'extérieur.

6. Procédé d'enduction selon l'une quelconque des revendications 1 à 5, **caractérisé en ce que** ledit processus pour établir le chemin d'application (P) établit le chemin d'application en un point d'intersection des axes de ladite pluralité de haubans (8) ou au voisinage dudit point d'intersection dans la partie de croisement dudit hauban (S).

7. Procédé d'enduction selon l'une quelconque des revendications 1 à 6, **caractérisé en ce que** ledit processus pour établir le chemin d'application (P) établit de façon identique les chemins d'application (P) d'une couche de revêtement (E1) où l'application est déjà finie et d'une couche de revêtement suivante (E2) sur ledit hauban (S).

8. Procédé d'enduction selon l'une quelconque des revendications 1 à 7, **caractérisé en ce que** ledit processus pour établir le chemin d'application (P) établit le chemin d'application de la couche de revêtement suivante (E2) de manière à ne pas chevaucher le chemin d'application (P) de la couche de revêtement (E1) où l'application est déjà finie sur ledit hauban (S) pour au moins une partie de celui-ci.

9. Procédé d'enduction selon l'une quelconque des revendications 1 à 8, **caractérisé en ce que** ledit processus d'application crée un espace (G) entre la surface de circonférence extérieure d'un mandrin (14) d'un support (10) qui tient ledit dispositif médical et la surface de circonférence intérieure du hauban (S) dudit dispositif médical au moins en un point de départ de l'application.

10. Procédé d'enduction selon l'une quelconque des revendications 1 à 9, **caractérisé en ce que** ledit processus d'application augmente la vitesse de déplacement pendant la section dans laquelle ladite tête d'application (30) passe plusieurs fois par rapport à la vitesse de déplacement pendant la section dans laquelle ladite tête d'application (30) ne passe qu'une fois.
